# EUROPEAN PATENT APPLICATION

(11) **EP 1 340 770 A1**
(43) Date of publication of application: **03.09.2003**
(21) Application number: 02290481.7
(22) Date of filing: 28.02.2002
(51) Int. Cl.: C08B 37/10, C07H 11/02

(54) **Nitro-derivatives of oligosaccharides**

(71) Applicant: Nicox S.A, 06906 Sophia Antipolis Cedex (FR)
(72) Inventor: Bolla, Manlio, 20138 Milano (IT); Monopoli, Angela, 20144 Milano (IT); Del Soldato, Piero, 20152 Monza (IT); Torri, Giangiacomo, 20133 Milano (IT); Naggi, Annamaria, 20025 Legnano (IT); Casu, Benito, 20133 Milano (IT)
(74) Representative: Barchielli, Giovanna

(57) **Abstract**

The invention concerns an oligosaccharide comprising uronic acid repeating units having an average molecular weight by weight comprised between 1,200 and 3,000 D and containing nitro groups -ONO₂ covalently bonded to the saccharide structure, and pharmaceutical compositions comprising it.

The oligosaccharide according to the invention is disclosed for use as a medicament, more specifically for use in the treatment and prevention of pathologies associated with disfunctions of haemostatic and cardiovascular systems.

## Description

### Summary of the Invention

The present invention concerns nitro-derivatives of oligosaccharides containing uronic acid repeating units, a process for the preparation thereof and the use thereof as a medicament.

### Background of the invention

Oligosaccharides containing units of uronic acid and, optionally hexosamine are known to be pharmacologically active compounds. One of the most important sources of these compounds is heparin.

Heparin is a mucopolysaccharide comprising repeating units formed of an uronic acid and a hexosamine residue. It can be obtained by extraction from beef, porcine, sheep, whale or other mammalian tissues with a solution of potassium acetate, alkaline ammonium sulfate and the like.

The commercial heparins have average molecular weight by weight ranging from about 10,000 to about 25,000 Da and patents and patent applications are known claiming different ways to achieve heparin fractions varying in molecular weight and pharmacological properties by physical separation, enzymatic, chemical or physical depolymerization.

Heparin fragments may be obtained by depolimerization with heparinase as described in US 4,847,338 or by depolimerization with nitrous acid which results in the formation of terminal units of anhydromannose (US 4,500,519 and US 4,351,938).

EP40144 and US 5,389,618 disclose depolymerization of heparin by beta-elimination from heparin ester.

WO 86/06729 discloses oligosaccharides, obtained by radical depolymerization of heparins, heparan sulfates, dermatansulfates, chondroitin sulfates and hyaluronic acid generated from a peracid or a peroxide in the presence of a catalyst selected from the group consisting of Cu⁺⁺, Fe⁺⁺, Cr⁺⁺⁺ and Cr₂O₇⁻⁻.

US 4,987,222 describes a process for the controlled preparation of low molecular weight glucosaminoglycans, by treating conventional high molecular weight glucosaminoglycans with gamma rays.

FR 2440376 and US 4692435 disclose the preparation of heparin fragments by solvent fractionation.

Another important method for the preparation of pharmacologically active oligosaccharides is by synthesis as described, for example, in EP 84 999, EP 300 099 and WO 99/36428, US 4,801,583, US 4,818,816 EP 165 134, . The patents disclose the synthesis of pentasaccharides which are similar to pentasaccharide sequences present in natural heparin and that show anti-thrombotic activity and especially potent anti-Xa activity without inactivating thrombin via AT - III.

High antithrombotic activity, high affinity to AT III and high anti - Xa activity are also the pharmacological properties of the oligosaccharides consisting of not more than 8 monosaccharides, described in US 4,401,662 and US 4,474,770.

US 4,351,938 teaches that heparin fragments having molecular weight of about 4,000 Da or less have higher therapeutic indices than natural heparin.

US 4,847,338 teaches that heparin fragments having molecular weight not greater than 2,300 Da present the capacity to inhibit complement activation.

EP 0 048 231 claims an oligosaccharide containing 4-8 monosaccharides, characterized by the presence of a 3-O-sulfated glucosamine and by a reducing end consisting of 2,5-anhydro-D-mannose, having selective anticoagulation activity.

Heparin oligosaccharides, having molecular weights of 1,000 to 3,500 Da, are used as active ingredients in pharmaceutical compositions having antithrombotic, thrombolytic and antiatherosclerotic activities (WO 90/04607).

WO 00/69444 discloses the use of glycosaminoglycans having an average molecular weight equal to 2,400 Da, for the preparation of pharmaceutical compositions suitable for the treatment of senile dementia.

The preparation of polysaccharides containing NO releasing groups has been recently proposed; however the direct introduction of nitro groups on heparin or heparin-like structures is not known.

US 6,261,594 discloses the preparation of chitosan-based polymeric nitric oxide donors obtained by introduction of a NONO group directly bonded to the backbone of the chitosan polymer.

J. E Saavedra et al, Bioorganic & Medicinal Chemistry Letters 10 (2000) 751-753, describe heparin/diazeniumdiolates conjugates that generate nitric oxide. However, the synthesis of these compounds is very complicated. In addition, the specific NO releasing group introduced in the heparin chain has various drawbacks and, consequently it is not suitable for pharmaceutical application.

### Description of the invention

The present invention provides oligosaccharides comprising uronic acid repeating units, having an average molecular weight by weight comprised between 1,200 and 3,000 D, sulfur content higher than 3 % by weight, and containing nitro groups -ONO₂ covalently bonded to the saccharide structure. Preferably, the sulfur content is comprised between 6 and 15% by weight.

In a first embodiment of the invention the oligosaccharides comprises heparin fragments.

In a further embodiment of the invention the oligosaccharides comprises at least 20%, preferably at least 50% of an oligosaccharide having Mw comprised between 1600 and 2000 D. This fraction is preferably obtained either by depolymerization of heparin or by synthesis.

The compositions according to the present invention present low or no anticoagulant activity and have the ability to bind several bioactive protein such FGF, Annexin II, patogen protein such TAT, adhesion protein such selectins, AP in amyloid plaque. This offer to this composition, a larger number of potential therapeutic applications in the treatment of cancer, viral and bacterial infections, Alzheimer's disease, and transplant rejection.

Furthermore, their anti-Xa activity and affinity for ATIII are function of their content of the unique pentasaccharide sequence AGA*IA (see formula II) that represents the antithrombin binding region (AT-BR) (Thunberg L. Backstrom G, Lindhal U, Carbohydr. Res 1982; 100:393-410).

Since early work (Casu et al. Biochem J., 197 1981 599-609) it was recognized that typical ¹³C NMR signals are associated with the 3-O-sulfated glucosamine residue (A*) characteristic of the active site for antithrombin, the C-2 signal of this residue has been used for the quantification of the AT-BR in heparins with different affinity for AT (Jonson EA et al. Carbohydr. Res. 51 1976 119-127). Signals associated with A* have been identified also in the ¹H NMR spectra ( Kusche et al. J. Biol. Chem 265 1990 7292-7300) and are currently used for quantification purposed also for LMWHs (Guerrini M. Seminars in thrombosis and hemostasis 27 5 2001 473-482). A* can range between 4 to 20 % on molar basis for the composition according to the present invention.

In a further embodiment, the invention relates to a composition comprising from 8 to 20%, preferably from 10 to 20% of A*. The composition presents high anti - Xa activity and affinity with AT-III. This is the result of the activity of natural or mimetic oligosaccharides (pentasaccharides) containing the sequence AGA*IA, increased by the effect of NO release due to the nitro groups introduced in the present invention. In fact, NO release increases the inhibition of platelets aggregation and confers to the compounds according to the invention a very high anti-clogging and anti-thrombotic activity together with a low risk of hemorrhagic events.

The expression heparin fragments indicates a polysaccharide prevalently formed of disaccharide repeating units formed of uronic acid and hexosamine, as indicated in Formula I

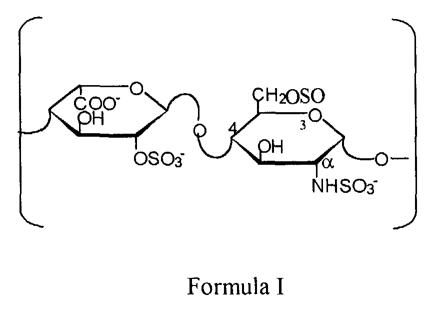

The nitro-derivatives of oligosaccharides according to the invention can be prepared according to different routes.

In a preferred embodiment, the oligomers are first prepared, either by depolymerization of natural heparin or by synthesys, and then subjected to nitration.

The depolymerization reaction can take place without modifying the hexosamine ring, for example by enzymatic hydrolysis with liase or hydrolase, by chemical depolymerization with mineral acids (e.g. sulfuric acid or hydrochloric acid), by demolition reaction of Smith or induced by gamma rays or beta elimination from an heparin ester.

The chemical depolymerization reaction by using nitrous acid results in the introduction of new functional groups: di- and oligosaccharides are obtained that present a terminal CHO which, by further reaction, gives rise to a new CH₂OH or a new amino group:

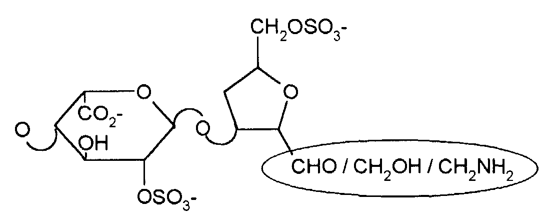

In another embodiment, the oligosaccharides of the present invention are chemically modified according to techniques well known in the art.

For example, N-sulfate groups of the hexosamino residue can be transformed into amino groups through desulfation reaction; the free amino groups can be N-acetylated; the sulfate groups of uronic acids can give rise to epoxy-groups through desulfation reaction; free amino groups and epoxy groups can be used to fix the polysaccharide to a polymeric support, as described in WO 99/27976.

It is also known the generation of a CH₂OH group by desulfation of the position 6 of hexosamine and another possible chemical modification is the supersulfation with adducts of pyridine - sulfur trioxide (K. Nagasawa; H. Uchiyama; N. Wajima, *Carbohydr. Res.* 158 (1986), 183-190; A. Ogamo, A. Metori; H. Uchiyama; K. Nagasawa, *Carbohydr. Res.* 193 (1989), 165-172; R.N. Rey; K.G. Ludwig-Baxter; A.S. Perlin, *Carbohydr. Res.* 210 (1991), 299-310) or with a mixture of sulfuric and chlorosulfonic acids (Naggi e al., *Biochem. Pharmacol.* 36, 1895-1900, 1987).

The nitration can take place in different positions of the saccharide units of heparin fragments. The preferred positions for the introduction of the nitro-containing groups are the following: CH₂OH in 6 of desulfated hexosamine, CHOH in 3 of hexosamine and of uronic acid, carboxy group in 6 of uronic acid, CHOH in 2 of desulfated uronic acid and NH₂ of desulfated hexosamine.

In a further embodiment of the invention, the nitro-derivatives of oligosaccharides are prepared by nitration of synthetic oligosaccharides. An example of useful synthetic oligosaccharide is given in formula II: 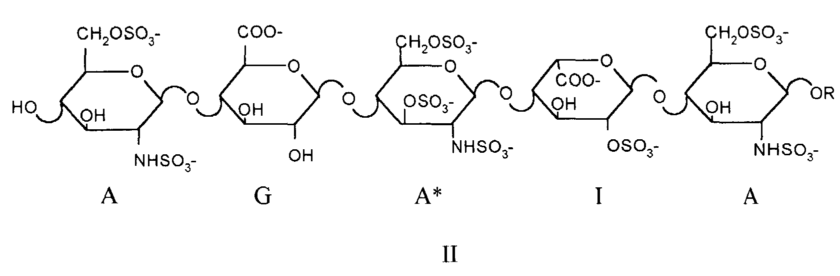 Wherein R is selected from the group consisting of H, C₁-C₄ alkyl. Preferably it is methyl.

Another example of synthetic oligosaccharide is given in formula III 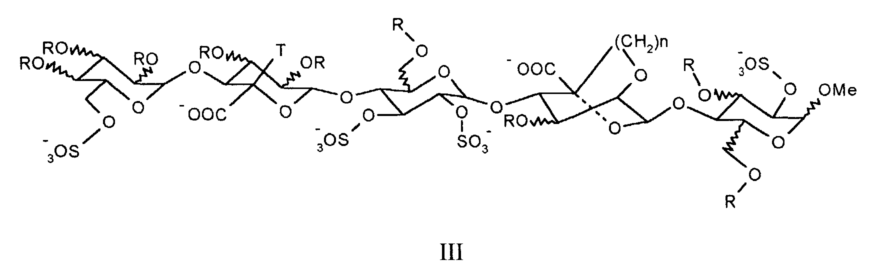 Wherein R is selected from the group consisting of H, C₁-C₄ alkyl. Preferably it is methyl.

The nitro group can be covalently bonded to the saccharide structure either directly (e.g. through nitration of a carbon of the saccharide unit) or through a divalent radical acting as a spacer between the saccharide unit and the nitro group. Suitable spacers are divalent radicals derived from C₂-C₂₀ aliphatic or aromatic hydrocarbons, ethers, polyethers, carboxylic acids or their derivatives, and the like.

A first path to the preparation of nitro-derivatives of oligosaccharides according to the invention is by direct nitration by means of a nitrating mixture. The reaction is preferably carried out by first preparing the nitrating mixture, wherein the previously dried oligosaccharide is added in small amounts under stirring. Examples of nitrating mixtures are sulfuric acid-nitric acid, phosphoric acid-nitric acid, acetic anhydride-nitric acid, nitrous oxide-sulfuric acid. Preferred mixtures are the mixture sulfuric acid-nitric acid, also called sulfo-nitric mixture and the mixture acetic anhydride-nitric acid. More preferred mixture is the mixture acetic anhydride-nitric acid.

Using the latter mixture, the molar ratio between acetic anhydride and nitric acid can vary in a broad range and is preferably comprised between 1:1 and 1:2, more preferably between 1:1.4 and 1:1.6. The amount of nitro groups per saccharide unit also varies considerably, and the ratio in equivalents between nitro groups and saccharide units is preferably comprised between 0.2 and 1.25, more preferably comprised between 0.30 and 0.80.

Another synthetic path for the synthesis of nitro-derivatives of oligosaccharides according to the invention is by haloacylation followed by nitration. In this case, it is possible to prepare first a VLMW-heparin containing N-desulfated hexosamine. The N-desulfated hexosamine is then reacted with a halo anhydride such as iodoacetic anhydride, obtaining the corresponding amide in position 2 of the hexosamine. The iodine atom is then substituted with a nitro group by reaction with a suitable nitrate. By using the suitable reaction conditions, it is possible to dose the amount of N-desulfation and, consequently, the amount of nitro groups introduced. Examples of suitable nitrates are AgNO₃ and NBu₄NO₃. It is possible to use anhydrides different from iodoacetic anhydride, such as the anhydride of a ω-halocarboxylic acid, obtaining in this way nitro-derivatives of oligosaccharides having a different spacer group. Preferred halogens are iodine and bromine. More preferred is iodine. Using heparin fragments and iodoacetic anhydride as starting materials, the reaction scheme is the following:

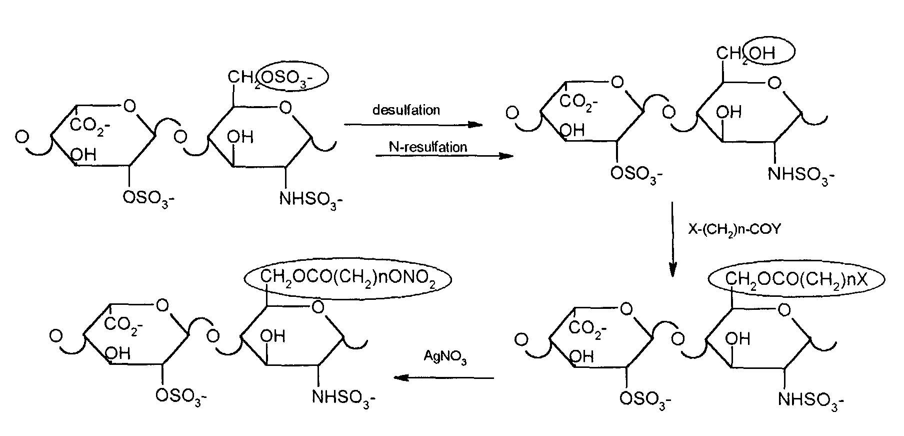

It is also possible to acylate the position 6 of desulfated hexosamine. In this case it is preferable to use compounds of formula X-(CH₂)ₙ-COY wherein X is chlorine, bromine, iodine or tosyl; preferably X is bromine; Y is chlorine, mercaptothiazole, mercaptobenzothiazole, mercaptobenzoxazole, p-nitrophenol. The reaction scheme is the following:

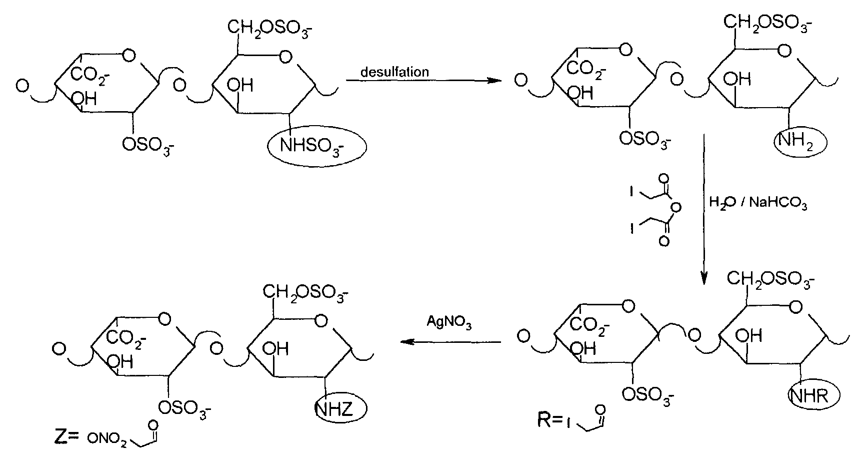

Another possible route of preparation of nitroderivatives is described in the following scheme, where the carboxyl group is activated by reaction with di cyclohexylcarbodiimide and then reacted with a compound of general formula X(CH₂)ₙY where Y is C1, Br, I, O-tosyl, ONO₂ and X is OH or NH₂:

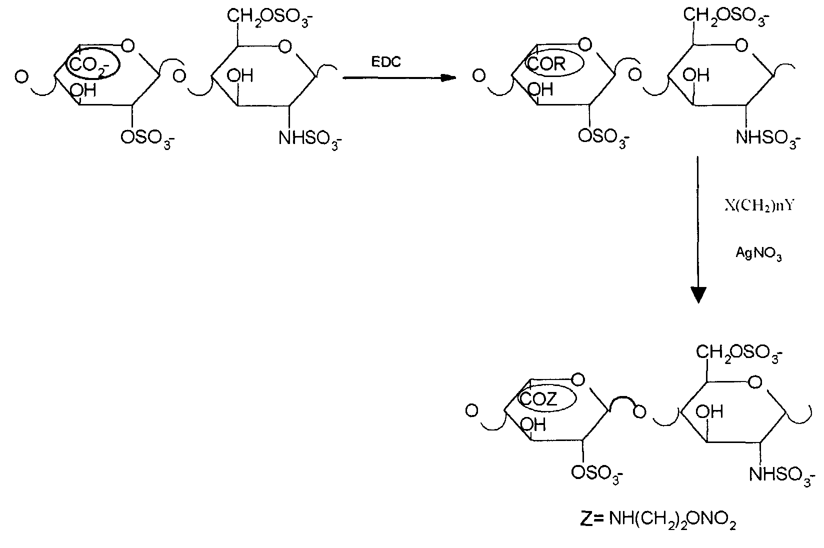

The compounds according to the present invention are useful in the treatment and in the prevention of pathologies associated with dysfunctions of the haemostatic and cardiovascular systems, such as arterial thromboembolism, venous thromboembolism, post surgical deep-vein thrombosis, pulmonary embolism, ischemic stroke, myocardial infarction and unstable angina, percutaneous transluminal coronary angioplasty (PCTA), atherosclerosis, endothelial dysfunctions of inflammatory nature such as flebites, and occurring with major pathologies such as diabetes or hypertension or caused by bacterial and viral infections.

The compounds according to the invention can be used in the preparation of pharmaceutical compositions in combination with the appropriate inert carrier, for topic, parenteral and oral use.

The formulation of therapeutic compositions is well known to persons skilled in this field. Suitable pharmaceutically acceptable carriers and/or diluents include any and all conventional solvents, dispersion media, fillers, solid carriers, aqueous solutions, coatings, antibacterial and antifungal agents, isotonic and absorption delaying agents, and the like. The use of such media and agents for pharmaceutically active substances is well known in the art, and it is described, by way of example, in Remington's Pharmaceutical Sciences, 18th Edition, Mack Publishing Company, Pennsylvania, US.

The nitro-derivatives of oligosaccharides according to the invention can optionally be used in combination with a compound that donates, transfers or releases nitric oxide elevates endogenous levels of nitric oxide, or is a substrate for nitric oxide synthase.

The compounds according to the present invention can also be used in combination with active substances normally used in the treatment of dysfunctions of the blood coagulating system and thrombotic system such as thrombolitic drugs, anticoagulant drugs, anti-platelet drugs, glycoprotein GP IIb/IIIa complex antagonists, thrombin antagonists. The active substance normally used in the treatment of dysfunctions of blood coagulating system and thrombotic system can be those known in the art including for example aspirin, dypiridamole, ticlopidine, clopidsrogrel, hirudine or new nitrooxyderivatives of NSAIDs, for example 2-(acetyloxy)benzoic acid-3-(nitroxymethyl)phenyl ester described in EP 759 899.

The present invention is now more deeply described through examples that must be considered explanatory but that are not intended to limit in any way the invention itself

### Experimental section

The reactions have been carried out on the following substrates:

### 1. VLMW heparin sodium salt

The analytical characteristics were the following:
Mw 2600 Mw/Mn 1.08
SO₃/COO⁻ (by ¹³C-NMR)= 2.3
A* = 6.36 % by ¹³C-NMR

This means that, considering as medium size of oligomeres present in this sample a octasaccharide, about 1 oligosaccharides on 4 has A* residue.
Elemental analysis: C: 22.37%; N: 2.03%;
atom of N per saccharide: 0.47

### 2. High charged fraction of Very Low Molecular Weight Heparin

It was prepared by ion exchange chromatography : 500 mg were loaded on a DEAE cellulose column (Ø= 2,5 cm h= 5 cm) and eluted by step with increasing concentration of NaCl (0.4; 0.8M, 1M, 2M) and NH₄Cl 0,05M.

Fraction 1M was desalted by size exclusion chromatography and the analytical characteristics were the following:
SO₃/COO⁻ (by ¹³C-NMR)= 2.65

Molar percentage of glucosamine and uronic acid residues via NMR
A* = 12.23 % by ¹³_{C-NMR}

This means that, considering as medium size of oligomeres present in this sample octasaccharides, about 1 oligosaccharide on 2 has A*.

### Example 1

### Preparation of nitroheparin via nitration of VLMW-heparin with nitro-acetic mixture

Nitroacetic mixture was prepared by dropping, over 45 minutes, 48 ml of cold acetic anhydride in 72 ml of nitric acid (90%) kept under stirring and cooled at -20°C in an N₂/acetone bath. At the end, 300 mg of VLMW-heparin were added. When the temperature was stabilized, N₂/acetone bath was changed with an ice bath; the mixture was stirred 3.5 hours at 0° C. 200 ml of a mixture diethyl ether - n-hexane 1:1 at -70°C in an N₂/acetone bath is dropped in the mixture, brought to -40°C. After 1 hour the sample is filtered avoiding dehydration, than the precipitate is dissolved in water, neutralized with a solution of diluited NaOH. The sample is concentrated through evaporation at a reduced pressure and at a low temperature. The product is purified by gel-filtration on Sephadex G10.

The product has the following characteristics:
Ethero correlate NMR spectra presence of a signal at : 83 ppm; 5,5 ppm
Elemental analysis: C: 3.18%; N: 1.00%;
   N per saccharide: 1.6
   ONO₂ per saccharide: 1.1

### Example 2

### Preparation of nitroheparin via nitration of high charged fraction of VLMW heparin with nitro-acetic mixture

Nitroacetic mixture was prepared by dropping, over 45 minutes, 32 ml of cold acetic anhydride in 48 ml of nitric acid (90%) kept under stirring and cooled at -20°C in an N₂/acetone bath. At the end, 200 mg of HEP were added. When the temperature was stabilized, N₂/acetone bath was changed with an ice bath; the mixture was stirred 4.5 hours at 0° C. 100 ml of a mixture diethyl ether - n-hexane 1:1 at -70°C in an N₂/acetone bath is dropped in the mixture, brought to -40°C. After 1 hour the sample is filtered until it is dry, the precipitate dissolved in water, neutralized with a solution of diluited NaOH. The sample is concentrated through evaporation at a reduced pressure and at a low temperature. The product is purified by gel-filtration on TSK.

The product has the following characteristics:
Ethero correlate NMR spectra presence of a signal at : 83 ppm; 5,5 ppm
Yield= 60 %

For the assessment of anticoagulant activity, two standard clotting tests were run using a ACL 7000 coagulometer, and all reagent kits from Instrumentation Laboratories, following the manifacturer instructions. Activated Partial Thromboplastin Time (aPTT) and Thrombin clotting Time (TT) were performed using plasma samples of 300 ul incubated with different concentration of compounds. Test were run automatically by the coagulometer. Data were plotted on computer to obtain concentration response curves or histogram plots, comparing control (vehicle treated), standard (reference compound) and treated (compound in this invention) samples.

The activity against the factor Xa (anti-Xa), a specific test intended to evaluate anti thrombotic activity was done as chromogenic test using the instrument and specific assay kits as above.

The anti platelet activity was explored by aggregometric assays on a Chrono-Log VS 560 instrument, using the nephelometric tecnique. Briefly, platelet rich plasma (PRP) was incubated with different concentrations of the compounds to analyse complete concentration response curves and then stimulated with a proaggregating agent (such as ADP).

To assess the NO release from the compounds of the invention, experiments were performed in presence or absence of direct inhibitors of soluble guanilyl cyclase, the enzyme responsible for the NO derived response.

## Claims

1. Oligosaccharides comprising uronic acid repeating units, having an average molecular weight by weight comprised between 1,200 and 3,000 D, sulfur content higher than 3 % by weight, **characterized in that** they contain nitro groups -ONO₂ covalently bonded to the saccharide structure.

2. Oligosaccharides according to claim 1 comprising at least 20% of fraction having Mw comprised between 1,600 and 2,000 D.

3. Oligosaccharides according to claim 1 comprising at least 8% of 3-O-sulfated glucosamine residue (A*).

4. Heparin according to claims 1-3, wherein equivalents ratio between nitro groups and saccharide units is comprised between 0.2 and 1.25.

5. Heparin according to claim 4, wherein the equivalents ratio between nitro groups and saccharide units is comprised between 0.30 and 0.80.

6. Heparin according to claims 1-5 wherein the nitro groups are introduced by reaction of one of the following positions: CH₂OH in 6 of desulfated hexosamine, CHOH in 3 of hexosamine and of uronic acid, carboxy group in 6 of uronic acid, CHOH in 2 of desulfated uronic acid and NH₂ of desulfated hexosamine.

7. Heparin according to claims 1-6, comprising at least 10% by weight of a pentasaccharide obtained by nitration of the following compound: 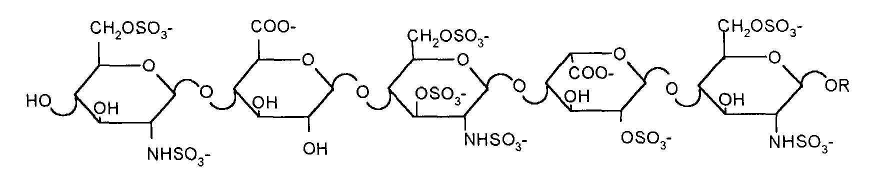 wherein R is selected from the group consisting of H, C₁-C₄ alkyl. Preferably it is methyl.

8. Heparin according to claims 7, comprising from 50 to 100% of pentasaccharide.

9. Heparin according to claims 1-8 wherein the nitro group is introduced by direct nitration using a nitrating mixture.

10. Heparin according to claims 1-8 wherein the nitro group is covalently bonded to the saccharide structure through a divalent radical R acting as a spacer between the polymeric chain and the nitro group, wherein R is a C₂-C₂₀ aliphatic or aromatic hydrocarbon, an ether, a polyether, a carboxylic acid or its derivatives.

11. Oligosaccharide according to claims 1-9 for use as a medicament.

12. Pharmaceutical compositions containing an oligosaccharide according to claims 1-9 admixed with pharmaceutically acceptable carriers.

13. Oligosaccharide according to claims 1-9 for use in the treatment and prevention of pathologies associated with dysfunctions of haemostatic and cardiovascular systems.

14. A composition comprising an oligosaccharide according to claims 1-9 and a compound that donates, transfers or releases nitric oxide elevates endogenous levels of nitric oxide, or is a substrate for nitric oxide synthase.

15. A composition comprising an oligosaccharide according to claims 1-9 in combination with a active substances normally used in the treatment of dysfunctions of the blood coagulating system and thrombotic system selected from the groups consisting of such thrombolitic drugs, anticoagulant drugs, anti-platelet drugs, glycoprotein GP IIb/IIIa complex antagonists, thrombin antagonists.
